# FASCICULE DE BREVET EUROPEEN

(11) **EP 0 370 336 B1**
(45) Date de publication et mention de la délivrance du brevet: **15.02.1995**
(21) Numéro de dépôt: 89120892.8
(22) Date de dépôt: 10.11.1989
(51) Int. Cl.: A61M 5/00

(54) **Dispositif monocoque polyfonctionnel pour le démontage et le stockage étanche d'instruments médicaux et chirurgicaux**
Einschalige Mehrzweckvorrichtung zum Demontieren und abgedichteten Lagern von medizinischen und chirurgischen Instrumenten
Single-shell polyfunctional device for disassembling and sealed storing medical and surgical instruments

(30) Priorité: 15.11.1988 FR 8815138
(43) Date de publication de la demande: 30.05.1990
(73) Titulaire: Germain, Bruno, F-69650 Saint-Germain au Mont d'Or (FR); Saby, Carnot, F-25870 Geneuille (FR)
(72) Inventeur: Germain, Bruno, F-69650 Saint-German au Mont d'Or (FR)
(74) Mandataire: Eggert, Hans-Gunther, Dr.

(56) Documents cités:
- WO-A-84/02674
- WO-A-88/08313
- DE-B- 2 601 512
- FR-A- 2 603 872
- US-A- 4 494 652

## Description

L'invention, concerne un dispositif monocoque pour le démontage et le stockage étanche d'instruments médicaux et chirurgicaux usagés, à jeter.

L'expression " INSTRUMENTS MEDICAUX ET CHIRURGICAUX" définira par la suite tous les instruments pour prélèver ou injecter un liquide et pour inciser tels que, par exemple, les vacutainers, les cataiters, aiguilles épicraniennes, aiguilles à pas de vis, aiguilles à ailettes, les dispositifs de perfusion, les lames de bistouris, les bistouris à usage unique, les scapels, et autres.

Depuis longtemps, le milieu médical utilise dans les hopitaux, cliniques, centres de soins, laboratoires d'analyse et autres lieux appropriés, non seulement des aiguilles hypodermiques ou équivalentes, telles que canules ou trocards pour prélever des liquides à analyser tel que le sang ou pour administrer des médicaments ou autres produits de traitements médicaux, mais aussi d'autres instruments médicaux et chirurgicaux pour prélever, injecter un liquide ou pour inciser.
Ces instruments dont il est fait une consommation importante, sont mis à la disposition du personnel médical sous une forme individuelle stérilisée et ensachée.
Après un premier usage, ces instruments ne sont pas restérilisés, mais sont détruits ou stockés en vue de leur destruction.
L'opération de stockage en vue de la destruction des instruments médicaux et chirurgicaux usagés doit s'accomplir d'une manière telle que le personnel médical ne risque pas une contamination par blessure accidentelle et que ces instruments usagés et stockés ne soient plus accessibles avant leur destruction.

C'est pourquoi, il s'est révèlé souhaitable que la séparation de l'instrument usagé et de son support, se fasse sans ou avec un minimum de contact manuel pour éviter les blessures accidentelles et les risques de contamination qui en résultent.

C'est pourquoi, il s'est révèlé également souhaitable d'isoler l'instrument usagé immédiatement après sa séparation d'avec son support afin d'éviter tout contact humain, accidentel ou intentionnel, qui pourrait conduire à une contamination ultérieure.

Tels qu'elles se révèlent, les publications de l'art antérieur ne concernent que pour l'essentiel, des dispositifs destinés à faciliter la manipulation et le stockage des aiguilles hypodermiques et équivalentes usagées.

Un premier type de dispositif dont un exemple est décrit dans le brevet FR-A-2,586,566 consiste en un support destiné à recevoir le fourreau proctecteur d'une aiguilles hypodermique stérile. Ce dispositif facilement préhensible est formé d'un corps lèger cylindrique, doté selon son axe, d'un réceptacle à commande bloquante pour le fourreau protecteur de l'aiguille et à l'extrêmité donnant accès au réceptacle, d'une corolle protectrice de la main tenant le corps léger cylindrique.
Ainsi l'aiguille hypodermique usagée est réintroduite dans son fourreau protecteur (placé en position bloquée dans le dispositif) sans risque de blessure pour l'opérateur médical tenant le dispositif.

Un premier inconvénient réside dans le fait que le dispositif exige deux manoeuvres pour l'opérateur médical, l'une étant la mise en place du fourreau contenant l'aiguille hypodermique stérile, puis l'extraction de l'aiguille stérile de son fourreau, l'autre étant la réintroduction de la dite aiguille usagée dans le fourreau, en utilisant la corolle protectrice de la main tenant le dispositif pour guider l'aiguille sans risque de blessure.

Un autre inconvénient, plus grave, apparaît à l'esprit quand il est perçu que le dispositif n'élimine pas l'aiguille usagée, ne permet que sa réintroduction dans le fourreau, et dès lors en laisse l'accès possible et facile avec tous les risques de blessures et de contamination.

Un dernier inconvénient se manifeste rédhibitoire dès lors que le dispositif par sa conception et sa structure, exclue son adaptation au démontage et stockage d'instruments médicaux et chirurgicaux autres que les aiguilles hypodermiques.

Un autre type de dispositif concerne les appareils destinés à tronquer les aiguilles hypodermiques usagées, et à en recueillir les fragments dans leur partie basse munie d'un conteneur amovible.

Un tel dispositif est décrit, par exemple, dans le Brevet US-A-4,255,996. Dans cette publication, le dispositif est formé de deux parties, l'une haute, l'autre basse, s'emboitant l'une dans l'autre pour constituer une enceinte close, mais démontable.
La partie haute est munie d'un orifice circulaire d'accès pour l'aiguille usagée, d'un diamètre supérieur à l'embout de la dite aiguille, orifice complèté par une fente de direction radiale, permettant la retenue verticale de l'aiguille usagée en position de coupe, par l'appui de l'embout sur les bords de la dite fente. La partie haute est également munie d'un mécanisme de coupe comportant deux lames parallèles pivotantes dotées d'une commande manuelle externe.
La partie basse est équipée d'un réceptacle amovible recueillant les fragments d'aiguilles usagées tronquées, Toutefois, ce dispositif intéressant par les résultats qu'il procure, présente aussi des inconvénients dont certains peuvent être considèrés comme importants.

Un premier inconvénient se manifeste par l'obligation pour l'opérateur médical, d'avoir un contact manuel avec l'aiguille pour la séparer de la seringue, avant ou après qu'elle ait été tronquée.

Un autre inconvénient se manifeste dès lors que l'orifice d'accès peut rester ouvert après la coupe et que l'enceinte est ouvrable par la séparation des parties haute et basse la formant. Ainsi le dispositif destiné à tronquer les aiguilles hypodermiques usagées n'est pas étanche et rend possible l'accès aux aiguilles tronquées avant leur destruction ultérieure, par le feu, par exemple.

L'ultime inconvénient apparaît dans le fait que ce type de dispositif est complètement inadapté et inadaptable par sa conception, au démontage d'instruments médicaux et chirurgicaux, autres que les aiguilles hypodermiques.

Un dernier type de dispositif concerne celui dont la vocation est de permettre la séparation de l'aiguille hypodermique usagée d'avec la seringue, son isolement immédiatement après la séparation en évitant tout contact humain accidentel qui pourrait provoquer une contamination de l'opérateur médical.

Un tel dispositif a été décrit, par exemple, dans WO-A-8 200 412, et consiste en un conteneur cylindrique dont la paroi supérieure est munie d'un orifice circulaire de diamètre inférieur à celui de l'embout de l'aiguille hypodermique. Cet orifice est placé à l'intersection de deux fentes en croix qui créent dans la paroi supérieure une zone flexible. L'aiguille usagée solidaire de la seringue est introduite en force dans l'orifice jusqu'à ce que l'embout traverse la paroi supérieure. Par traction sur la seringue, l'aiguille tombe au fond du conteneur.

Mais ce dispositif présente l'inconvénient majeur d'être toujours ouvert, et de ce fait, de ne pas être étanche, et dès lors de rendre possible l'accès aux aiguilles usagées ou autres instruments équivalents se trouvant dans le conteneur.

Un autre dispositif, relevant de ce dernier type, décrit dans la demande européenne de brevet EP-A-0,123,247, consiste en un récipient collecteur d'aiguilles hypodermiques usagées, en forme de flacon dont l'ouverture est munie d'une coiffe ayant un orifice de forme triangulaire très spéciale pour permettre l'engagement de l'aiguille solidaire de la seringue, la séparation par traction de la seringue et la chute de l'aiguille.
Dès lors que l'aiguille a été séparée et stockée dans le récipient collecteur, l'orifice triangulaire est aveuglé par un mouvement de rotation de la coiffe, interdisant l'accès du récipient collecteur d'aiguilles.

Bien que ce dispositif évite apparemment le contact manuel avec l'aiguille au cours de sa séparation, il apparaît être doté de certains désavantages majeurs qui en limitent l'usage.

Un premier désavantage réside dans le fait que ce dispositif n'est pas aisément utilisable quand il n'est pas à poste fixe, car l'ouverture de l'orifice triangulaire d'accès s'effectuant par rotation de la coiffe, provoque également la rotation du dispositif s'il n'est pas solidement maintenu en position.
Dès lors, le personnel médical se trouve confronté à deux alternatives. Oubien se déplacer du patient jusqu'au dispositif de stockage à poste fixe, la seringue munie de son aiguille usagée dans une main, tandis que l'autre main saisie la coiffe, lui imprime un mouvement de rotation jusqu'à l'ouverture de l'orifice triangulaire d'accès. Oubien pose la seringue munie de son aiguille usagée, saisie à deux mains le dispositif de stockage, l'une entrainant la rotation de la coiffe, l'autre bloquant le dispositif.

Un autre désavantage se manifeste dans le fait que l'orifice d'accès de forme triangulaire ne s'adapte pas au diamètre de l'embout de l'aiguille à séparer de la seringue. De ce fait, pour que l'aiguille soit retenue par le dit orifice, le manipulateur, après introduction de l'aiguille solidaire de la seringue dans la zone la plus large de l'orifice triangulaire, déplace la seringue selon une translation horizontale jusqu'à ce que l'aiguille soit bloquée par le rétrécissement de l'orifice triangulaire.
Il en découle que l'on ne peut éviter le blocage occasionnel d'une aiguille dans l'orifice triangulaire, nécessitant son dégagement manuel, avec les risques de blessures et de contamination.

Un troisième désavantage réside dans le volume du dispositif qui rend relativement encombrant et d'un transport peu aisé puisqu'il doit être assujetti à un poste fixe, tel que table roulante de soins, par exemple.

Un dernier désavantage est constitué par la structure même du dispositif, qui se manifeste inadapté au démontage d'instruments médicaux et chirurgicaux autres que les aiguilles hypodermiques.

Dans la demande de brevet FR-A-2 603 872, la demanderesse s'est attachée à éliminer les inconvénients précités, grâce à un dispositif monofonctionnel amélioré, étanche, de caractèristiques dimensionelles restreintes pour être facilement transportable dans une poche et aisément manipulable parce que tenant dans une main, dans lequel l'aiguille usagée puisse être introduite facilement, et séparée de la seringue sans contact humain direct, et dans lequel l'aiguille collectée est définitivement isolée.

Selon la demande de brevet précitée, le dispositif comprend :
- A) Un boitier réceptacle muni d'un orifice d'accès pour les aiguilles.
- B) A l'intérieur du boitier réceptacle, et au-dessous de l'orifice d'accès, un écran plan mobile qui obture le dit orifice, mais qui le libère à la demande par déplacement.
- C) Un organe de commande de l'écran plan mobile.

Toutefois, tel qu'il est structuré, ce dispositif est seulement adapté au démontage et au stockage d'aiguilles hypodermiques usagées ou équivalentes, mais ne permet pas le démontage et le stockage étanche de l'ensemble des instruments médicaux et chirurgicaux usagés, et plus particulièrement les instruments d'incision, tels que lames de bistouris.
Ainsi, le dispositif précité présente l'inconvénient d'être monofonctionnel.

Il en est de même avec le dispositif de la DE-B-26 01 512 qui décrit un dispositif monocoque pour le démontage et le stockage étanche d'instruments médicaux qui se compose d'un boitier-réceptacle indéformable doté de deux orifices d'accès, de moyens d'obturation formés par deux écrans plans-mobiles qui obturent les dits orifices et les libèrent à la demande, d'un organe de commande des écrans plans-mobiles.

Dès lors la demanderesse vise à éliminer les inconvénients précités, en perfectionnant le dispositif monofonctionnel, en le dotant des moyens nouveaux lui permettant le démontage et le stockage étanche de tous les instruments médicaux et chirurgicaux usagés à jeter, tels que définis antérieurement.

L'objet de l'invention est donc un dispositif monocoque pour le démontage et le stockage étanche d'instruments médicaux qui se compose
a) d'un boitier-réceptacle indéformable (1) doté de deux orifices d'accès (2) et (3),
b) de moyens d'obturation formés par deux écrans plans-mobiles (4) et (5) qui obturent les dits orifices et les libèrent à la demande,
c) d'un organe de commande (6) des écrans plans-mobiles,
caractérisé en ce que les orifices sont disposés sur une même face du boitier-réceptacle et que leurs sections sont différenciées dans leurs profils en vue d'assurer l'introduction et/ou la séparation des parties dangereuses des différents types d'outils médicaux et chirurgicaux.

Le boitier-réceptacle au contraire des dispositifs de l'art antérieur, peut avoir des dimensions telles qu'il est facilement appréhendé par la main libre de l'opérateur médical et tenu en position stable pendant que l'autre main tient l'instrument médical ou chirurgical usagé à éliminer.

Le boitier-réceptacle dont le section peut être indifférement polygonale ou circulaire est de type monocoque, c'est à dire que tous les moyens nécessaires eu fonctionnement du dispositif y sont enfermés. Ce boitier est généralement constitué de deux demi-coquilles moulées qui permettent avant leur assemblage, d'introduire tous les moyens intervenant dans la réalisation et le fonctionnement du dispositif selon l'invention.
L'assemblage ultérieur des deux demi-coquilles, par soudage, par exemple, rend le boitier-réceptacle étanche.
Les orifices d'accès, généralement placés sur une même face du boitier-réceptacle, jouent la même fonction de démontage et d'élimination, mais sont dotés de moyens différents conformés aux instruments médicaux et chirurgicaux à traiter.

L'un des orifices d'accès, plus spécialement destiné au démontage non seulement des aiguilles hypodermiques et équivalentes, mais également des aiguilles type vacutainer, (aiguilles à embout vissant cannelé, sur la seringue) des cataiters, des aiguilles épicraniennes, des aiguilles à ailettes, des dispositifs de perfusion et autres dispositifs à usage unique, tels que bistouris à usage unique, injectable monobloc (type monojet) disposent comme moyen, d'un évidement dont la forme géomètrique polyvalente peut être circulaire ou proche d'un cercle, hémicirculaire, élliptique, émiélliptique, ou encore être la combinaison de l'une des formes géomètriques précitées avec une forme carrée, rectangulaire, ou encore polygonale génèrant des formes polygonales curvilignes.

L'autre orifice d'accès, plus précisément destiné au démontage des lames de bistouris et scapels, dispose comme moyen, d'un évidement dont la forme géomètrique peut être carrée, rectangulaire ou polygonale.
Les orifices d'accès dont sont munis le boitier-réceptacle sont généralement dotés d'une pièce tronconique externe servant de guide pour l'instrument médical et/ou chirurgical à traiter.

La périphérie interne de l'un au moins des orifices d'accès peut être dotée en tout ou partie de cannelure qui favorisent par blocage, la préhension et le démontage de la partie à éliminer de l'instrument médical et/ou chirurgical.

A l'intérieur du boitier-réceptacle, dans l'axe et au-dessous des orifices d'accès, sont placés des écrans plans-mobiles dont le mouvement de translation ou de rotation est commandé de l'extérieur du boitier par l'opérateur médical. Ces écrans plans-mobiles obturent les orifices d'accès quand le dispositif selon l'invention est au repos.

Chaque écran plan-mobile est généralement formé d'une lame unique rigide pouvant comporter un évidement de forme géomètrique, telle que carrée, rectangulaire, circulaire, hémicirculaire, élliptique, hémiélliptique, éventuellement combinées entre elles, ou encore en forme de T, L, U.

D'une manière très préférencielle, l'évidement de chaque écran plan mobile dispose d'une dimension au moins égale à celle de l'orifice d'accès le concernant

L'évidment de l'un au moins des écrans plans-mobiles est placé en concordance axiale avec l'orifice d'accès correspondant, quand le dit écran mis en mouvement libère le dit orifice d'accès.

L'un au moins des orifices d'accès, et l'un au moins des évidements des écrans plans-mobiles peuvent être dotés sur tout ou partie de leur périphérie interne, de cannelures facilitant la préhension et le blocage de la partie de l'instrument médical et/ou chirurgical à démonter, et donc, à séparer, la séparation étant d'autant plus aisée que l'orifice d'accès et l'évidement de l'écran plan-mobile sont simultanément munis des dites cannelures.

Chaque écran plan-mobile est rendu solidaire d'un seul organe de commande de son mouvement provoqué par le contact avec l'un des doigts de la main tenant le dispositif selon l'invention.

Dès lors qu'ils sont en mouvement, l'un au moins des écrans plans-mobiles libère en l'ouvrant l'orifice d'accès correspondant, permettant l'introduction dans le boitier-réceptacle, de la partie usagée de l'instrument médical ou chirurgical à démonter et à éliminer. Oubien l'introduction dans le boitier-réceptacle de l'instrument médical ou chirurgical à usage unique.

L'organe de commande des écrans plans-mobiles est muni d'un moyen de rappel dans la position d'obturation de l'orifice d'accès, quand il est libèré du contact digital, rendant automatique et sécurisante la fermeture du dispositif après usage.

Pour favoriser encore la sécurité de l'usager, l'organe de commande des écrans plans-mobiles peut également entraîner le mouvement d'un écran supplémentaire d'obturation (par exemple, souple ou monté sur pivots) se trouvant généralement au-dessous des écrans mobiles. Cet écran supplémentaire, quand il est au repos, complète l'obturation étanche de l'un au moins des orifices, et libère l'accès des dits orifices quand l'organe de commande est sollicité.

Le dispositif selon l'invention peut être doté à l'intérieur du boitier-réceptacle, de moyens anti-retour, empêchant définitivement l'accès, même accidentel, aux instruments médicaux et chirurgicaux usagés et stockés, et rendant étanche le dit boitier, en y maintenant les liquides provenant des instruments démontés, même lors d'un retournement du boitier-réceptacle et de l'ouverture intempestive des orifices.

Ces moyens anti-retours sont constitués, par exemple, d'écrans fixes de formes planes, tronconiques, troncpyramidales, munis d'un orifice de section circulaire, polygonale, élliptique ou autres éventuellement dotés d'un embout tubulaire, cylindrique, tronconique troncpyramidale ou autres, permettant l'introduction des instruments usagés et interdisant leur retour.

De plus, le dispositif selon l'invention peut être doté d'une paroi verticale interne créant deux zones de stockage sélectif, pour les divers instruments usagés à éliminer.

Enfin, les orifices d'accès et plus précisément celui destiné au démontage des lames de bistouri et scapels peuvent être dotés d'un sas, permettant d'augmenter encore la sécurité de l'usager, les moyens d'ouverture et de fermeture des dits sas ainsi que les moyens de commande restant les mêmes que ceux précités.

Les matériaux mis en oeuvre pour la réalisation de dispositif selon l'invention, sont en général réalisés en matières polymères thermo-formables, tels que, par exemple, les polyolifines, les polyamides, les polyesters, les polymers halogènés.

Ces matières polymères peuvent recevoir des charges constituées par des matériaux minéraux pulvérulents, tels que carbonate de calcium, sulfate de calcium, kaolins, dioxyde de titane, talc, alumine, mais aussi par des fibres de verre, fibres céramiques, fibres de carbone.

Ces matières polymères peuvent recevoir également des adjuvents très divers, tels que par exemple, des colorants, des agents bioxides, des agents antiphotons, etc...

Aussi, et contrairement à l'art antérieur, le dispositif selon l'invention, présente de multiples avantages grâce à la conception du boitier-réceptacle monocoque. Bien plus, sa forme et ses dimensions permettent à l'opérateur médical de la manier aisément, d'une seule main, tandis que l'autre tient l'instrument médical ou chirurgical usagé à démonter ou à éliminer.

Enfin, le retour automatique dans la position d'obturation des orifices d'accès après introduction d'instruments médicaux ou chirurgicaux usagés, évite l'ultime geste de sécurité que le personnel médical peut omettre quand de tels dispositifs doivent être clos manuellement.

L'invention sera mieux comprise grâce à la description illustrative et non limitative faite à partir des dessins annexés.

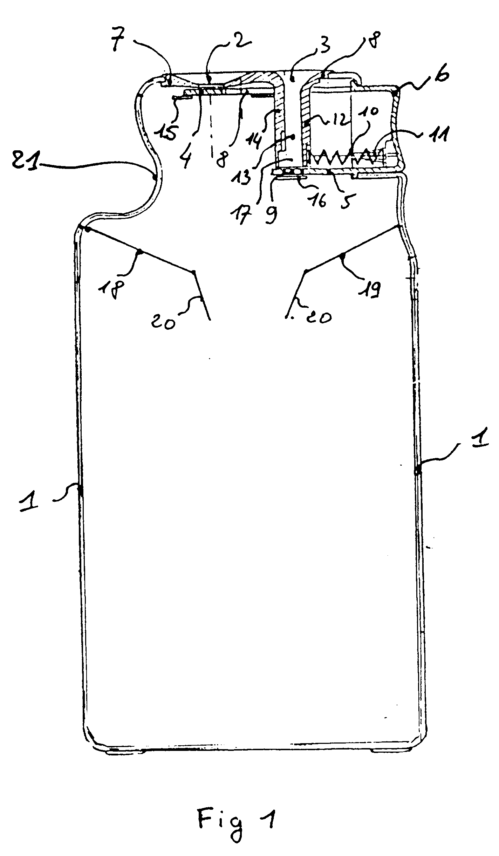

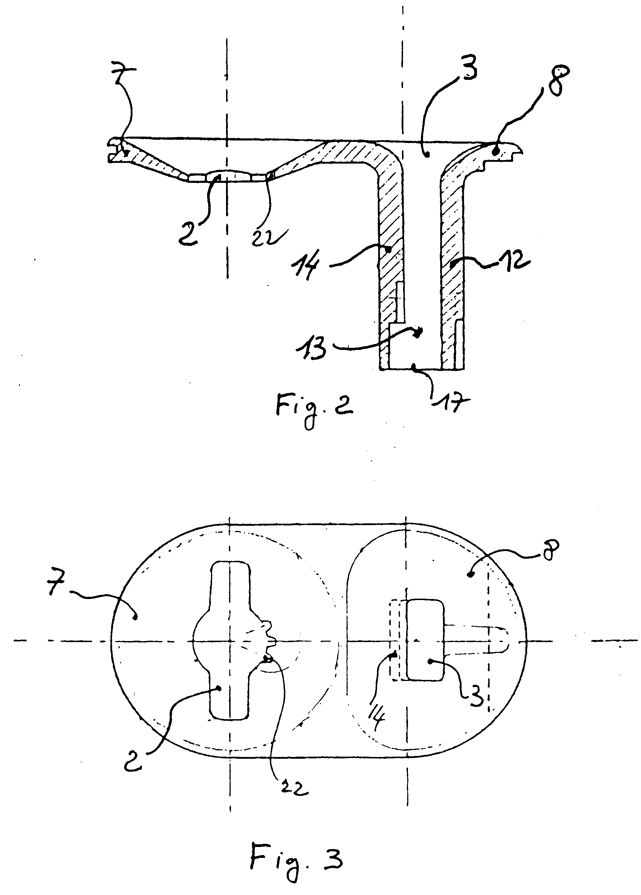

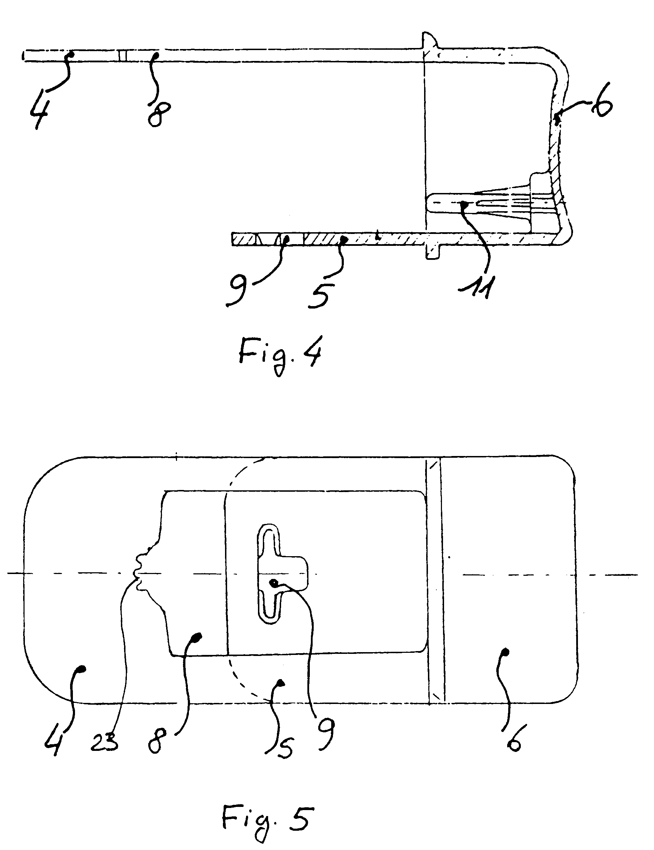

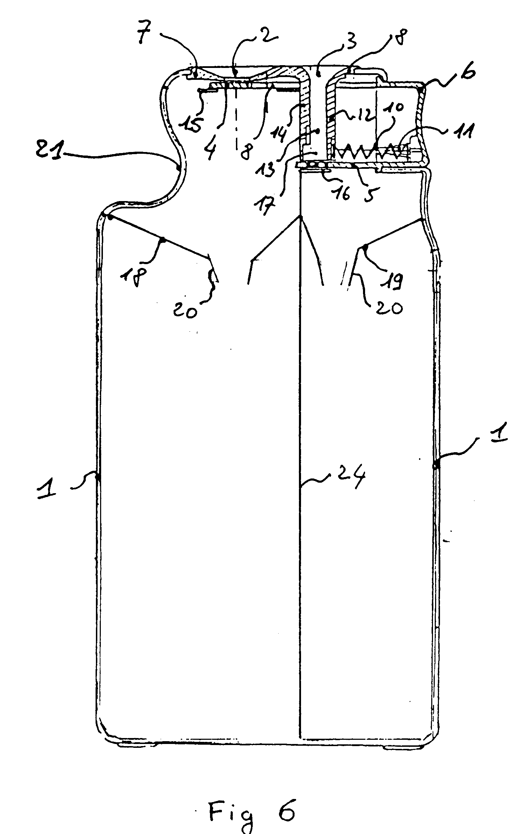

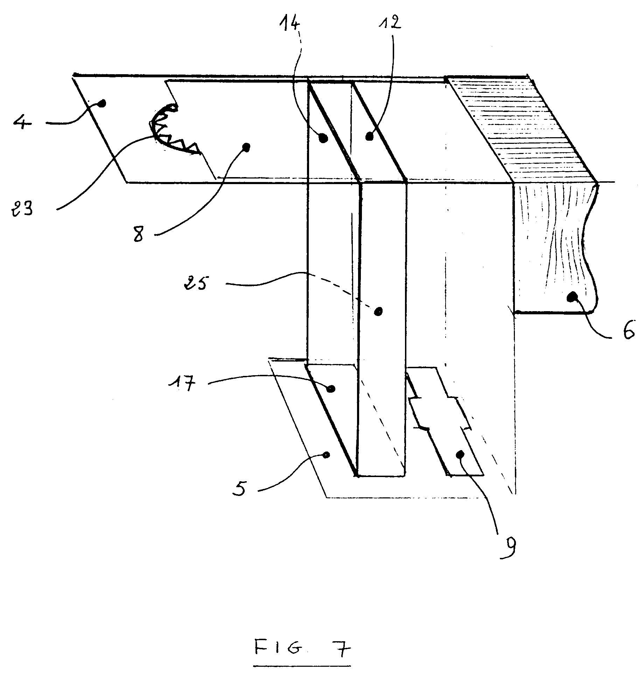

La figure (1) est une coupe longitudinale en élévation d'un dispositif réalisé selon l'invention.

La figure (2) est une coupe longitudinale en élévation des orifices d'accès.

La figure (3) est une vue par dessus, des orifices d'accès.

La figure (4) est une coupe longitudinale en élévation des écrans plans-mobiles et l'organe de commande du mouvement des dits écrans.

La figure (5) est une vue par dessus des écrans plans-mobiles.

La figure (6) est une coupe longitudinale en élévation du dispositif selon l'invention, doté d'une paroi interne créant deux zones de stockage.

La figure (7) est une vue en perspective d'un sas mettant en oeuvre les écrans plans-mobiles et l'organe de commande du mouvement des dits écrans.

Selon la figure (1), le dispositif pour le démontage et/ou le stockage avant destruction d'instruments médicaux et chirurgicaux usagés, se compose d'un boitier-réceptacle (1) muni d'un premier orifice d'accès (2) et d'un deuxième orifice d'accès (3).

Au-dessous des orifices d'accès (2) et (3) et à l'intérieur du boitier-réceptacle (1) sont placés des écrans plans-mobiles (4) et (5) et un organe de commande (6) des dits écrans mobiles.

Le boitier-réceptacle (1) est formé de deux demi-coquilles moulées et assemblées par soudage en fin de montage.

Les orifices d'accès (2) et (3) sont munis de pièces tronconiques externes (7) et (8) servant de guide pour l'introduction dans le boitier des instruments médicaux et chirurgicaux usagés.

L'écran plan-mobile (4) qui, au repos, obture l'orifice d'accès est muni d'un évidement (8) dont les dimensions sont au moins identiques à celles de l'orifice (2).

De même, l'écran plan-mobile (5) est doté d'un évidement (9) de forme géomètrique et de dimensions adaptées à celles de l'orifice d'accès (3).

Les écrans plans-mobiles (4) et (5) sont situés dans des plans parallèles, et sont rendus solidaires l'un de l'autre par l' intermédiaire de l'organe de commande (6) de leur mouvement.

L'organe de commande (6) du mouvement des écrans plans-mobiles est muni d'un moyen de rappel (10) constitué par un ressort de compression dont l'une des extrêmités est contrôlée par l'ergot (11) solidaire de l'organe de commande (6), tandis que l'autre extrêmité prend appui en butée sur la paroi (12) du prolongement (13) de l'orifice (3).

Les extrêmités libres des écrans plans-mobiles (4) et (5) sont guidées lors de leur mouvement par les points d'appui (15) et (16).

Au-dessous de l'écran plan-mobile (5) peut se trouver un moyen mobile (non-représenté) d'obturation complémentaire du prolongement (13) de l'orifice (3), le dit moyen mobile d'obturation complémentaire étant également solidaire de l'organe (6) de commande de mouvement et libèrant l'extrêmité (17) du prolongement (13) de l'orifice (3) quand le dispositif est mis en service.

La figure (1) montre également la présence d'un moyen anti-retour dont la fonction est d'empècher la sortie accidentelle d'un instrument médical ou chirurgical usagé lors d'une manipulation maladroite, en particulier si l'utilisateur était tenté d'inverser le sens du dispositif selon l'invention, tout en maintenant volontairement les orifices d'accès (2) et (3) ouverts par pression sur l'organe (6) de commande de mouvement.

Le moyen anti-retour précité est formé des parois planes (18) et (19) prolongées d'une tubulure (20) à paroi tronconique ou cylindrique.

Enfin, la figure (1) révèle la présence dans la partie supérieure du dispositif du rétreint (21) permettant une excellente préhension du dispositif selon l'invention, par l'utilisateur.

Selon les figures (2) et (3), l'orifice d'accès (2) muni de la pièce tronconique externe (7) servant de guide pour l'introduction dans le dispositif, des instruments médicaux et chirurgicaux usagés est doté sur une partie de la périphérie interne, de cannelures (22) favorisant le blocage de l'instrument usagé au moment du démontage de la partie à séparer, en vue de sa destruction ultérieure.

Quant à l'orifice d'accès (3) muni de la pièce tronconique externe (8) jouant le rôle de guide pour l'introduction des instruments médicaux et chirurgicaux qui ne peuvent être traités par l'orifice d'accès (2), le dit orifice (3) se prolonge par les parois (12) et (14) selon le prolongement (13) qui débouche par l'ouverture (17) dans la zone de stockage du dispositif selon l'invention.

Dans leur réalisation industrielle, les deux orifices d'accès (2) et (3), les pièces tronconiques (7) et (8) et le prolongement (13) constituent une seule pièce mécanique qui peut être réalisée facilement par moulage, par exemple, d'un matériau polymère.

Les figures (4) et (5) montrent les écrans plans-mobiles (4) et (5) et l'organe de commande du mouvement (6).
Les écrans plans-mobiles (4) et (5) situés dans des plans parallèle sont rendus solidaires l'un de l'autre par l'organe de commande (6) de leur mouvement. Chaque écran plan-mobile est doté d'un évidement (8) pour l'écran (4), et (9) pour l'écran (5), dont les formes géomètriques sont dans le cas présent adaptées aux empreintes des instruments médicaux et chirurgicaux.

L'évidement (8) est doté sur la périphérie interne, de cannelures (23) favorisant le blocage de l'instrument usagé au moment du démontage de la partie de l'instrument médical ou chirurgical à séparer, en vue de sa destruction.

La figure (6) montre le dispositif pour le démontage et/ou le stockage avant destruction d'instruments médicaux et chirurgicaux usagés muni des mêmes moyens que ceux de la figure (1), mais doté en plus d'une paroi verticale interne (24) créant deux zones de stockage sélectif.

La figure (7) montre un sas (25) équipant l'orifice d'accès (3) le dit sas étant doté des écrans plans-mobiles (4) et (5) rendus solidaires l'un de l'autre par l'organe de commande (6) de leur mouvement. Le sas (25) est formé par le prolongement (13) des parois (12) et (14) de l'orifice (3) jusqu'à déboucher par l'ouverture (17) dans la zone de stockage, dès lors que l'évidement (9) de l'écran (5) est mis en concordance avec la zone inférieure dudit sas.

En pratique, le dispositif selon l'invention tel que décrit est mis en oeuvre de la manière suivante :
L'opérateur médical tenant d'une main l'instrument médical ou chirurgical usagé, saisit le dispositif selon l'invention, de l'autre main, et présente l'instrument usagé à traiter devant l'orifice le mieux adapté au dit instrument.

Dès lors, d'un doigt de la main tenant le dispositif fait pression sur l'organe de commande du mouvement des écrans plans-mobiles. Le mouvement est alors transmis aux écrans plans-mobiles (4) et (5)

Les évidements (8) et (9), de ce fait placés en concordance avec les orifices d'accès (2) et (3), l'instrument médical ou chirurgical usagé est alors introduit dans l'orifice d'accès choisi et traverse l'évidement de l'écran plan-mobile correspondant.

L'opérateur médical relâche alors, la pression sur l'organe (6). Les évidements (8) et (9) des écrans plans-mobiles (4) et (5) sous l'action du ressort (10) tendent à revenir à leur position initiale, et de ce fait, bloquent la partie usagée de l'instrument médical ou chirurgical à éliminer. Cette dite partie étant dès lors aisément séparée par une faible traction ou une simple rotation. La partie usagée du dit instrument tombe dans le boitier-réceptacle (1). Les écrans plans-mobiles libèrés reviennent à leur position initiale de repos et d'obturation sous l'action du ressort (10) et ferment donc à nouveau les orifices d'accès.

## Revendications

1. Dispositif monocoque pour le démontage et le stockage étanche d'instruments médicaux qui se compose
a) d'un boitier-réceptacle indéformable (1) doté de deux orifices d'accès (2) et (3),
b) de moyens d'obturation formés par deux écrans plans-mobiles (4) et (5) qui obturent les dits orifices et les libèrent à la demande,
c) d'un organe de commande (6) des écrans plans-mobiles,
caractérisé en ce que les orifices sont disposés sur une même face du boitier-réceptacle et que leurs sections sont différenciées dans leurs profils en vue d'assurer l'introduction et/ou la séparation des parties dangereuses des différents types d'outils médicaux et chirurgicaux.

2. Dispositif selon la revendication 1 caractérisé par le fait que l'un au moins des orifices d'accès (2) et (3) est muni en tout ou partie de cannelures.

3. Dispositif selon les revendications 1 et 2 caractérisé par le fait que les orifices d'accès (2) et (3) sont munis d'une pièce tronconique externe de guidage de l'instrument médical et chirurgical usagé.

4. Dispositif selon l'une quelconque des revendications 1 à 3 caractérisé par le fait que l'un des orifices a un évidement de forme géométrique du type circulaire, hémicirculaire, élliptique, hémiélliptique.

5. Dispositif selon la revendication 4 caractérisé par le fait que l'un des orifices a un évidement de forme géométrique, carrée, rectangulaire, polygonale, combinée avec l'une des formes circulaire, hémicirculaire, élliptique, hémiélliptique.

6. Dispositif selon l'une quelconque des revendications 1 à 5 caractérisé par le fait que l'autre orifice a un évidement de forme géométrique, carrée, rectangulaire, polygonale.

7. Dispositif selon l'une quelconque des revendications 1 à 6 caractérisé par le fait que les écrars plans-mobiles (4) et (5) sont formés de deux lames rigides parallèles entre elles.

8. Dispositif selon l'une quelconque des revendications 1 à 7 caractérisé par le fait que l'évidement (8) et (9) de chaque écran plan-mobile a une forme géométrique carrée, rectangulaire, circulaire, hémicirculaire, elliptique, hémiélliptique, en forme de L, T, U, combinée ou non deux à deux.

9. Dispositif selon l'une quelconque des revendications 1 à 8 caractérisé par le fait que l'évidement (8) ou (9) de chaque écran plan-mobile (4) et (5) dispose d'une dimension au moins égale à celle de l'orifice d'accès le concernant (2) et (3).

10. Dispositif selon l'une quelconque des revendications 1 à 9 caractérisé par le fait que l'un au moins des évidements (8) et (9) des écrans plans-mobiles (4) et (5) est muni en tout ou partie de cannelures.

11. Dispositif selon l'une quelconque des revendications 1 à 10 caractérisé par le fait que l'organe de commande (6) est un poussoir doté d'un moyen de rappel (10).

12. Dispositif selon la revendication 11 caractérisé par le fait que le moyen de rappel est un ressort.

13. Dispositif selon l'une quelconque des revendications 1 à 12 caractérisé en ce que l'organe de commande entraîne également un écran supplémentaire d'obturation se trouvant au-dessous des écrans plans-mobiles.

14. Dispositif selon l'une quelconque des revendications 1 à 13 caractérisé en ce qu'il comporte une paroi interne verticale (24) créant deux zones de stockage sélectif.

15. Dispositif selon l'une quelconque des revendications 1 à 14 caractérisé en ce que l'un au moins des orifices d'accès est doté d'un sas de sécurité (25).

16. Dispositif selon l'une quelconque des revendications 1 à 15 caractérisé par le fait que le boitier-réceptacle (1) est muni d'un moyen interne anti-retour (19) des instruments usagés stockés.

17. Dispositif selon l'une quelconque des revendications 1 à 16 caractérisé par le fait que le moyer interne anti-retour (19) est formé d'écrans fixes de formes planes, tronconiques, troncpyramidales.

18. Dispositif selon la revendication 17 caractérisé par le fait que le moyen anti-retour est muni d'un orifice (20).

19. Dispositif selon la revendication 18 caractérisé par le fait que l'orifice (20) est un embout tubulaire.

## Claims

1. Single-casing appliance for removal and sealed storage of medical instruments comprising
a) a dimensionally stable receptacle-casing (1) which has two openings (2) and (3),
b) means of closure formed by two flat, mobile screen-covers (4) and (5) which close and open said openings as required,
c) an operating member (6) for operating flat movable screen covers,
characterized in that openings are disposed on a single surface of a receptacle-casing and their sections are different in profile in order to ensure introduction and/or separation of dangerous part of different types of medical and surgical instruments.

2. Appliance in accordance with claim 1, characterized in that at least one of openings (2) and (3) is either partly or fully provided with fluting.

3. Appliance in accordance with claims 1 and 2, characterized in that openings (2) and (3) are provided with an external tapered member for guiding used medical and surgical instruments.

4. Appliance in accordance with any one of claims 1 to 3, characterized in that one of openings has a circular, hemispherical, elliptical or semi-elliptical recess.

5. Appliance in accordance with claim 4, characterized in that one of openings has a recess which is square, rectangular or polygonal, combined with a form which is circular, hemispherical, elliptical or semi-elliptical.

6. Appliance in accordance with any one of claims 1 to 5, characterized in that another opening has a square, rectangular or polygonal form.

7. Appliance in accordance with any one of claims 1 to 6, characterized in that flat, movable screen covers (4) and (5) are formed of two parallel non-flexible sheets.

8. Appliance in accordance with any one of claims 1 to 7, characterized in that a recess (8) and (9) of each of two flat, movable screen covers has a square, rectangular, circular, hemispherical, elliptical or semi-elliptical, in L-, T- or U-forms, either in combinations of twos or not in combinations of twos.

9. Appliance in accordance with any one of claims 1 to 8, characterized in that a recess (8) or (9) of each of two flat, movable screen covers (4) and (5) is at least as large as an opening (2) and (3) it is associated with.

10. Appliance in accordance with any one of claims 1 to 9, characterized in that at least one of recesses (8) and (9) of flat, movable screen covers (4) and (5) is fully or partly provided with fluting.

11. Appliance in accordance with any one of claims 1 to 10, characterized in that an operating member (6) is a push-button with a resetting means (10).

12. Appliance in accordance with claim 11, characterized in that a resetting means concerned is a spring.

13. Appliance in accordance with with any one of claims 1 to 12, characterized in that an operating means concerned also involves a supplementary screen cover placed below flat, movable screen covers.

14. Appliance in accordance with with any one of claims 1 to 13, characterized in that it has an internal, vertical wall (24) which creates two selective storage areas.

15. Appliance in accordance with with any one of claims 1 to 14, characterized in that at least one of openings has a safety airlock (25).

16. Appliance in accordance with with any one of claims 1 to 15, characterized in that a casing-receptacle (1) is provided with an internal reverse lock means (19) to stop return of used instruments stored.

17. Appliance in accordance with with any one of claims 1 to 16, characterized in that a reverse lock means (19) is formed by fixed screens which have a flat or a truncated conical or a truncated pyramidal form.

18. Appliance in accordance with claim 17, characterized in that a reverse lock means concerned is provided with an opening (20).

19. Appliance in accordance with claim 18, characterized in that an opening (20) is a tubular joining piece.

## Patentansprüche

1. Einschalige Vorrichtung zur Demontage und dichten Aufbewahrung von medizinischen Instrumenten, bestehend aus:
a) einem formbeständigen Aufnahmebehälter (1), versehen mit zwei Zugangsöffnungen (2) und (3),
b) Verschlußvorrichtungen, gebildet aus zwei ebenen beweglichen Platten (4) und (5), welche die Öffnungen verschließen und sie bei Bedarf freigeben,
c) einem Betätigungsorgan (6) für die ebenen beweglichen Platten, dadurch gekennzeichnet, daß sich die Öffnungen auf ein und derselben Seite des Aufnahmebehälters befinden und daß ihre Querschnitte sich im Profil unterscheiden, um die Einbringung und/oder Trennung von gefährlichen Teilen unterschiedlicher medizinischer und chirurgischer Instrumententypen zu gewährleisten.

2. Vorrichtung nach Anspruch 1, dadurch gekennzeichnet, daß mindestens eine der Zugangsöffnungen (2) und (3) ganz oder teilweise mit Rillen versehen ist.

3. Vorrichtung nach den Ansprüchen 1 und 2, dadurch gekennzeichnet, daß die Zugangsöffnungen (2) und (3) mit einer externen kegelstumpfartigen Führung für das benutzte medizinische und chirurgische Instrument versehen sind.

4. Vorrichtung nach einem der Ansprüche 1 bis 3, dadurch gekennzeichnet, daß eine der Öffnungen eine Vertiefung mit kreisförmiger, halbkreisförmiger, elliptischer oder semi-elliptischer Geometrie aufweist.

5. Vorrichtung nach Anspruch 4, dadurch gekennzeichnet, daß eine der Öffnungen eine Vertiefung hat, die eine quadratische, rechteckage, vieleckige Geometrie kombiniert mit einer kreisförmigen, halbkreisförmigen, elliptischen oder semi-elliptischen Form aufweist.

6. Vorrichtung nach einem der Ansprüche 1 bis 5, dadurch gekennzeichnet, daß die andere Öffnung eine Vertiefung hat, die eine quadratische, rechteckige, vieleckige Geometrie aufweist.

7. Vorrichtung nach einem der Ansprüche 1 bis 6, dadurch gekennzeichnet, daß die ebenen beweglichen Platten (4) und (5) aus zwei zueinander parallel stehenden starren Streifen gebildet werden.

8. Vorrichtung nach einem der Ansprüche 1 bis 7, dadurch gekennzeichnet, daß die Vertiefung (8) und (9) einer jeden ebenen beweglichen Platte eine quadratische, rechteckige, kreisförmige, halbkreisförmige, elliptische, semi-elliptische Geometrie, in Form eines L, T, oder U aufweist, wobei jeweils zwei kombiniert sein können oder nicht.

9. Vorrichtung nach einem der Ansprüche 1 bis 8, dadurch gekennzeichnet, daß die Vertiefung (8) oder (9) einer jeden ebenen beweglichen Platte (4) und (5) eine Abmessung aufweist, die mindestens der der betreffenden Zugangsöffnung (2) und (3) entspricht.

10. Vorrichtung nach einem der Ansprüche 1 bis 9, dadurch gekennzeichnet, daß mindestens eine der Vertiefungen (8) und (9) der ebenen beweglichen Platten (4) und (5) ganz oder teilweise mit Rillen versehen ist.

11. Vorrichtung nach einem der Ansprüche 1 bis 10, dadurch gekennzeichnet, daß das Betätigungsorgan (6) eine Druckvorrichtung ist, die mit einem Rückstellmechanismus (10) versehen ist.

12. Vorrichtung nach Anspruch 11, dadurch gekennzeichnet, daß der Rückstellmechanismus eine Feder ist.

13. Vorrichtung nach einem der Ansprüche 1 bis 12 dadurch gekennzeichnet, daß das Betätigungsorgan gleichfalls eine zusätzliche Verschlußplatte mitnimmt, die sich unterhalb der ebenen beweglichen Platten befindet.

14. Vorrichtung nach einem der Ansprüche 1 bis 13, dadurch gekennzeichnet, daß sie eine innere vertikale Wand (24) aufweist, wodurch zwei getrennte Unterbringungszonen gebildet werden.

15. Vorrichtung nach einem der Ansprüche 1 bis 14, dadurch gekennzeichnet, daß mindestens eine der Zugangsöffnungen mit einer Sicherheitsschleuse (25) versehen ist.

16. Vorrichtung nach einem der Ansprüche 1 bis 15, dadurch gekennzeichnet, daß der Aufnahmebehälter (1) mit einer internen Entnahmesperre (19) für die benutzten, aufbewahrten Instrumente versehen ist.

17. Vorrichtung nach einem der Ansprüche 1 bis 16, dadurch gekennzeichnet, daß die interne Entnahmesperre (19) aus festen Platten mit ebener, kegelstumpfartiger oder pyramidenstumpfartiger Form gebildet ist.

18. Vorrichtung nach Anspruch 17, dadurch gekennzeichnet, daß die Entnahmesperre mit einer Öffnung (20) versehen ist.

19. Vorrichtung nach Anspruch 18, dadurch gekennzeichnet, daß die Öffnung (20) aus einem röhrenförmigen Ansatzstück besteht.
